**Europäisches Patentamt**

**European Patent Office**

**Office européen des brevets**

(11) Publication number : **0 410 664 B1**

# EUROPEAN PATENT SPECIFICATION

(45) Date of publication of patent specification :
31.03.93 Bulletin 93/13

(51) Int. Cl.⁵ : **A61K 9/00**

(21) Application number : **90308031.5**

(22) Date of filing : **23.07.90**

(54) **Composite bolus and its use.**

(30) Priority : **26.07.89 GB 8917063**

(43) Date of publication of application :
**30.01.91 Bulletin 91/05**

(45) Publication of the grant of the patent :
**31.03.93 Bulletin 93/13**

(84) Designated Contracting States :
**AT BE CH DE DK ES FR GB GR IT LI LU NL SE**

(56) References cited :
**DE-A- 2 735 754**
**GB-A- 2 109 665**
**GB-A- 2 111 388**

(73) Proprietor : **BORDEN (UK) LIMITED**
**Rownhams Road North Baddesley**
**Southampton S052 9ZB (GB)**

(72) Inventor : **Johnson, Alan Edward**
**57 Scarisbrick Road**
**Rainford, St. Helens, WA11 8JN (GB)**
Inventor : **Greengrass, Jane**
**7 West View**
**Ormskirk, Lancashire (GB)**

(74) Representative : **Wilkinson, Stephen John et al**
**Stevens, Hewlett & Perkins 1 St. Augustine's**
**Place**
**Bristol BS1 4UD (GB)**

## Description

This invention relates to a process for the manufacture of a composite bolus for sustained release of a sparingly soluble organic active material in the reticulo-rumen of an animal and to the use of such a bolus in the animal.

Various devices have been proposed for sustained release of an active substance such as a drug or dietary supplement. Systems in which the active substance is contained within a polymer are known and use various mechanisms to achieve the desired sustained release. However, it is difficult to engineer the degree of control required and there are problems with polymer toxicity potential.

Systems in which the active substance is contained within an inorganic material, such as a soluble phosphate glass, are also known. Such systems are unsuitable for most organic active substances because the high temperatures required to form a bolus can affect the structure of the active substance.

Attempts have been made to produce composite boluses in which active substances are retained in a structure containing both inorganic and organic components. GB 2109665 is an example of such a composite bolus. The active substance is held in a matrix of organic cellulosic polymer which also contains particles of phosphate glass. Dissolution of the phosphate glass allows fluid to penetrate the bolus and reach the active substance. Such a bolus is said to be suitable for delayed release of the active material and sustained release from such a composite would not be easily controlled, particularly if a hydrophobic sparingly soluble organic active substance is to be released. Such a bolus would also have disadvantageous release characteristics in situations where the active substance is supplied as a sodium salt which would have its solubility impaired due to the common ion effect in the presence of soluble sodium containing glasses. GB 2 109 665 does not give detailed disclosure of the manufacturing method for the composite bolus, but it does say that the particle size of the glass should be smaller than the size of the active substance so that the particles can be bound together like a mortar with the cellulosic material. It also says that the body may be formed by pressing.

An alternative release mechanism for boluses containing a sparingly soluble active substance is simple abrasion. European patent 97507 describes a bolus having a core of compressed particles enclosed in a rigid, insoluble, brittle, open-ended tube. The bolus is abraded at its exposed end and the rate of disintegration of the end of the tube controls the release rate. Journal Article No. 4105 of the Oklahoma Agricultural Experimental Station, Stillwater discusses boluses which use such an abrasion technique for their release mechanism and notes that the use of two or more boluses gives a higher release rate. It also notes that such boluses are subject to irregular degradation in the reticulum.

It is an object of the present invention to provide a bolus which is sufficiently reliable to be used with sparingly soluble organic active substances that are toxic if administered too quickly and ineffective if administered too slowly. Furthermore the bolus should be capable of operating with this reliability over a sustained period.

According to the present invention a composite bolus for sustained release of a sparingly soluble organic active material in the reticulo-rumen of an animal characterised in that it is produced by mixing together an adhesive comprising one or more insoluble thermosetting resins, which are highly substantive to glass when cured, with an equal or greater weight of a dispersion medium comprising one or more hydrophilic organic liquids which are miscible with or dissolve the uncured adhesive and phase separate from the adhesive when it is cured, to form a liquid binder; mixing the binder so formed with glass or other inorganic particles and particles of sparingly soluble active material to form a cake, the ratio of binder to particles being selected to ensure the presence of voids in the cake; and curing the adhesive to phase separate it and bond together the glass particles to form a rigid bolus.

When using other inactive inorganic materials, for instance sand, in place of glass an adhesive must be chosen that is substantive to the inorganic material when cured; suitable adhesives can be determined by comparatively simple trials.

The cake may be pressed around a ballast weight before cure or cured cake may be fixed to the ballast weight.

The glass may be a phosphate glass or may be a more durable glass such as 'E' glass or soda-lime glass. The adhesive may be one or more epoxy resins such as bisphenol diglycidyl ether cured with an aliphatic polyamine, such as diethylene triamine. Adhesives that are liquid at 25°C are preferred. Epi sulphides may be used as adhesives.

The organic liquid may include a liquid which is immiscible with water, such as castor oil or may include a liquid which is miscible with water, such as polyoxyethylene (1000) sorbitan mono-oleate, available from Atlas Chemical Industries Inc. under the trade mark TWEEN 80, polyethylene glycol 1000 sorbitan mono-laurate, available from Atlas Chemical Industries Inc. under the trade mark TWEEN 20, or polyethylene glycol (400) dioleate (known as PEG 400 dioleate).

The glass may be coated with a silane to reduce permeation of water into the bolus.

The glass may be a phosphate glass with a $P_2O_5$ content of greater than 50 wt% and an $Al_2O_3$ content of greater than 5 wt%. Advantageously a glass particle size of 450 micrometres or more preferably less than 200 micrometres is used in conjunction with an average particle size of active substance of approximately 45 micrometres. Also in accordance with the present invention a method for the sustained supply of an active substance to a ruminant animal comprises administering simultaneously two or more boluses prepared by the method according to the invention to the reticulum of the animal, with the proviso that therapeutical treatments are excluded. Preferably two boluses are so administered and desirably the boluses are of equal size and weight.

The invention will now be further described by way of example only and with reference to the accompanying drawings, of which:-

Figure 1 is a graphic representation of the loss of weight of a pair of boluses due to mutual abrasion in a sheep over a period of weeks.

Figure 2 is a graphical representation of the rate of weight loss of a pair of boluses in a cow, the boluses containing a different drug from the boluses represented in figure 1.

Figure 3 is a graphical representation of the results of a roller abrasion test showing the residual weight of boluses containing various resin mixes.

Figure 4 is a graphical representation of the loss of weight of boluses, made from durable glass, within a cow over a period of weeks.

Figure 5 is a graphical representation of the results of a roller abrasion test showing the residual weight of boluses containing various ratios of bonding : non-bonding material.

Figure 6 is a graphical representation of the loss of weight of boluses in a cow and the release of a drug from the boluses. Registered trademarks as used in the following are acknowledged as such.

Example 1

An epoxide resin comprising bisphenol A diglycidyl ether cured with diethylene triamine was mixed with various organic liquids at room temperature and then cured at 50°C. Dodecanol was found to be immiscible with the epoxide. Silicone oil gave rise to an unstable dispersion when cold mixed with the epoxide. Reoplex 400, available from Ciba-Geigy under the trade mark REOPLEX 400, was readily miscible with the epoxide, but did not phase separate on cure. Castor oil formed a stable dispersion at room temperature, which became homogeneous on warming and phase separated satisfactorily on cure. Tween 80, which is miscible with water, was readily miscible with the epoxide and phase separated satisfactorily on cure. Polyethylene glycol 400 dioleate was also readily miscible with the epoxide at room temperature and gave rise to satisfactory phase separation on curing.

Example 2

Having identified combinations of insoluble adhesive and organic liquids that phase separated satisfactorily on cure at 50°C a bolus was prepared for testing by the following method. A one kilogramme batch of glass was melted from standard raw materials and cast as cullet which was then crushed in a Glen-Cresten hammer mill using 500 micrometre and 200 micrometre screens. For this example we used glass having a weight percent composition of $P_2O_5$ 55%, $Al_2O_3$ 11%, MgO 8%, CaO 1.9%, $Na_2O$ 23%, $K_2O$ 0.9%. This glass has a theoretical solubility in distilled water at 39°C of 8 milligrammes per gramme per day when the glass is in the form of grains 710-1000 micrometres in diameter. As soon as possible after removal from the hammer mill the glass grains were sieved to the required particle size of less than 75 micrometres.

Meanwhile the resin mix was prepared by combining 24.4 parts by weight of Epikote 828, available from Shell Chemical Company under the trade mark EPIKOTE 828, a bisphenol A diglycidyl ether, with 2.44 parts of diethylene triamine (DET) and 73.2 parts by weight of Tween 80. For this example the Epikote 828 and the Tween 80 were mixed in a beaker and the diethylene triamine added from a pipette.

Batches were then made up as follows. 3 grammes of the resin mix was then mixed with 5 grammes of a drug for administration to a ruminant animal supplied by the manufacturer in powdered form and 25 grammes of the glass composition given above. The mixtures were hand mixed for 5 minutes and then both were loaded into an agate ball mill and rotated for approximately 30 minutes. 6.6 gramme discs of 18 mm diameter were pressed at 0.5 tonnes and a bolus made by gluing discs to each end of a cylindrical mild steel ballast weight with epoxy resin. This gave a bolus density of 2.82 grammes per cubic centimetre when the ballast weight was 11.4g. It is important that void spaces are present in the bolus. To ensure that voids are present the mixture must be non-hydrostatically pressed, in other words the particulate phase must not be under hydrostatic pressure. By pressing in this way the interstitial space between the particulate phases is only partially filled with binder and the matrix is still porous to liquid. The residual void space is an essential feature in pressing stable preforms. The applicants have found that as a rough rule-of-thumb guide a resin mix content of approximately 40% of that required to fill the void space is a suitable amount of resin to use to obtain a bolus of suitable viscosity, that is a bolus that will hold its shape. The amount of resin required can be easily determined for the glass constituent; glass of mass M will, when crushed take up a volume $V^1$, before being crushed it would have had a volume V, therefore a volume of resin $V^1$-V will fill up the voids in the crushed glass. As this is only a rule-of-thumb the errors inherent in measuring the volume of a crushed mass of glass are negligible. The amount of resin required to fill the voids in the drug component must be estimated.

Example 3

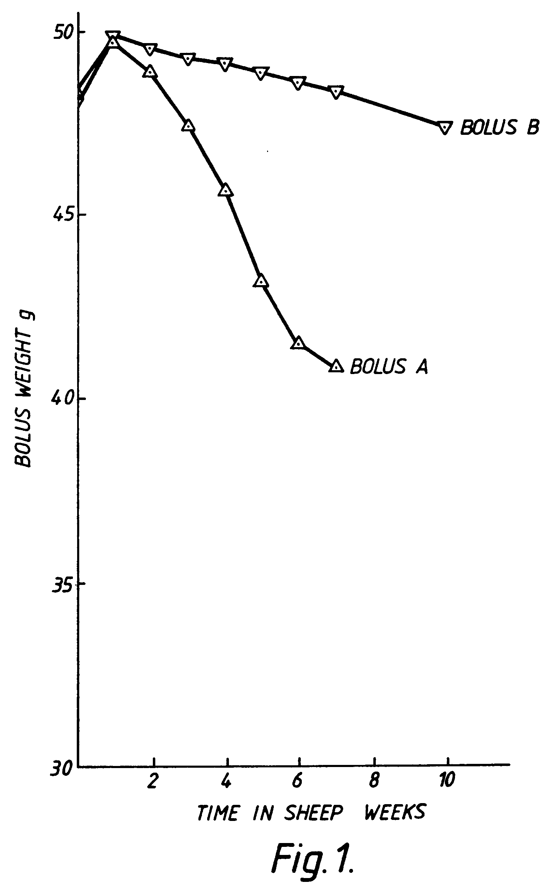

A pair of identical boluses of the type manufactured by the process described in Example 2 were inserted into the reticulum of a sheep. Two sheep were used for the tests. Into one sheep a pair of boluses having composition A was inserted and into the other sheep a pair of boluses having composition B was inserted. The constituents and proportions of constituents in each composition are given in Table 1. The drug used was Closantel, available from Janssen Pharmaceutica under the trade mark CLOSANTEL. The phosphate glass was that described in Example 2 and the resin mix was the same as that described for Example 2, with the weight percentages being varied according to Table 1. Figure 1 shows the loss of weight of the boluses due to mutual abrasion within the sheep over a period of several weeks. Initially the boluses are seen to gain in weight, this is due to uptake of water into the void spaces in the bolus. The weight of the boluses was determined by removing them from a fistulated animal , drying them with a paper towel and weighing them. Although previous work had reported that abrasion of pairs of boluses gave rise to uneven weight loss, it was observed that the combined weight loss of a pair of boluses gave rise to a satisfactory loss in weight irrespective of whether one bolus was contributing more than the other at any one time.

Table 1

| Bolus | A (w/w) | B (w/w) |
|---|---|---|
| Epikote 828 | 2.2 | 3.3 |
| Tween 20 | 6.6 | 5.5 |
| DET | 0.2 | 0.3 |
| Closantel | 15.2 | 15.2 |
| Glass | 75.8 | 75.8 |

It will be seen from Figure 1 that bolus pair A lost weight considerably faster than bolus pair B. Bolus A had a higher proportion of organic liquid to adhesive and it is apparent that within limits the abrasion rate can be altered by varying this ratio.

Theory predicted that particles of Closantel would be available to the animal once released into the reticulum from the abrading bolus. Blood samples were taken periodically and tested for Closantel to confirm this theory. Bolus A gave rise to a steadily increasing level of Closantel in the animal's blood over the period of release, whereas bolus B gave rise to a roughly constant, lower, level of Closantel in the animal's blood. It can be concluded that by altering the rate of release it is possible to administer a drug to the animal to maintain a given, approximately constant, level of drug in the animal's blood stream.

Example 4

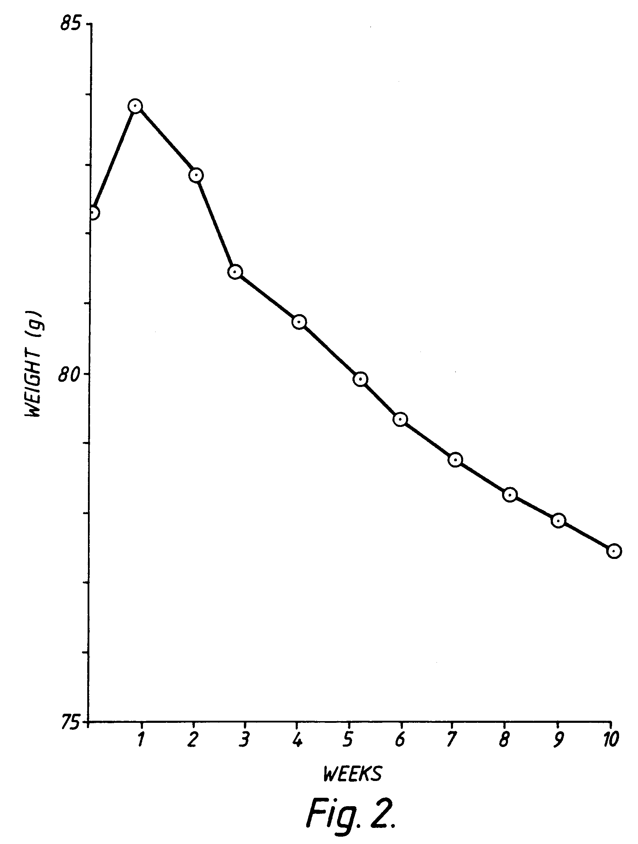

Boluses prepared in accordance with the method given for Example 2, with Monensin, supplied by Sigma Chemical Company, replacing the Closantel, were administered to a fistulated cow. Figure 2 shows the rate of weight loss profile for these boluses which is similar to the profile shown in Figure 1.

4

Example 5

Resins were prepared in accordance with the method given for Example 2 with varying ratios of Tween 80 to Epikote 828 to produce epoxide weight percentages in the resin of 25%, 20%, 16.67% and 14.33%. The resins were then formed into boluses in accordance with the method given in Example 2 except that the drug was omitted. As the drug release rate may be controlled by the abrasion rate of the boluses, only the abrasion rates were measured in this and the following examples. The abrasion rates in fistulated sheep for the various epoxide percentages in the resin are shown in Table 2.

TABLE 2

| % EPOXIDE | ABRASION RATE $(gd^{-1})$ |
|---|---|
| 25 | 0.055 |
| 20 | 0.06 |
| 16.67 | 0.09 |
| 14.33 | 0.120 |

The 25% and 14.33% abrasion rates were estimated due to experimental difficulties. However, it does seem likely that there is a rapid increase in abrasion rate with decreasing epoxide content.

Example 6

For some of the experimental work an artificial reticulum was used in a Roller Abrasion Test (R.A.T.). The R.A.T. involves rotating a bolus in a glass jar containing water and chips of ethyl vinyl acetate co-polymer The glass jar is periodically agitated and the bolus weighed at intervals of 24 hours and the erosion debris removed from the jar. The R.A.T. appears to give a greater abrasion rate than that which would occur in an animal due to the nature of the co-polymer chips. Qualitatively, however, the R.A.T. appears to be a valid test.

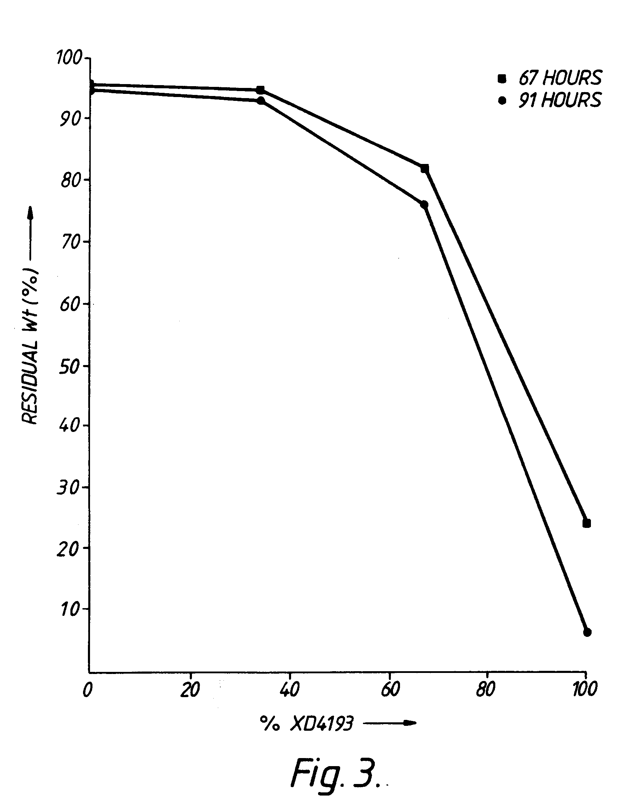

The effect of substituting an alternative epoxide in the resin mix was assessed by the R.A.T. test. Two resin premixes were prepared in accordance with the method of Example 2 using a 50% epoxide content and using Tween 20 in place of Tween 80 as the non-bonding phase. In the first resin Epikote 828 was used with 10% DET, whilst in the second resin the epoxide was XD 4193, available from Ciba-Geigy, with 5% DET. Four sets of boluses were prepared in accordance with the method of Example 2 using a resin premix to filler weight ratio of 1:10 using the same glass as in Example 2. The four sets of boluses each contained different percentages of the two resins 0, 33.33, 66.66 and 100% by weight respectively, of the second resin the balance of the resin component being the first resin. Figure 3 shows the residual weight of boluses from each of the four sets (as a percentage of their initial weight) after testing for two time periods, 67 hours and 91 hours in the R.A.T.. It can be seen that as the percentage of the softer XD 4193 epoxide increases the abrasion rate also increases.

Example 7

Two resin mixes were prepared using 47.6 parts by weight of Epikote 828 and 4.8 parts by weight of DET. In the first mix 47.6 parts by weight of Tween 80 was used, in the second the same amount of castor oil was used in place of the Tween 80. Each of the resins was mixed with a glass having a weight percent composition of $SiO_2$ 72.5%, $Al_2O_3$ 0.4% MgO 3.3%, CaO 9.8%, $Na_2O$ 13.7% and $K_2O$ 0.1%. This is a very durable glass. The glass was prepared in accordance with the method of Example 2 and mixed in a resin:glass weight ratio of 1:10. The first, Tween 80, glass/resin mixture was pressed at 1 tonne to produce a 65.75 gramme bolus with a mean density of 3.14 grammes per cubic centimetre. The second, castor oil, glass/resin mixture was similarly pressed to produce a 65.58 gramme bolus with a mean density of 3.19 grammes per cubic centimetre. Both

EP 0 410 664 B1

the boluses were made by pressing a steel disc into a steel tube and gluing the moulded composite into the cavity between the steel disc and the steel tube.

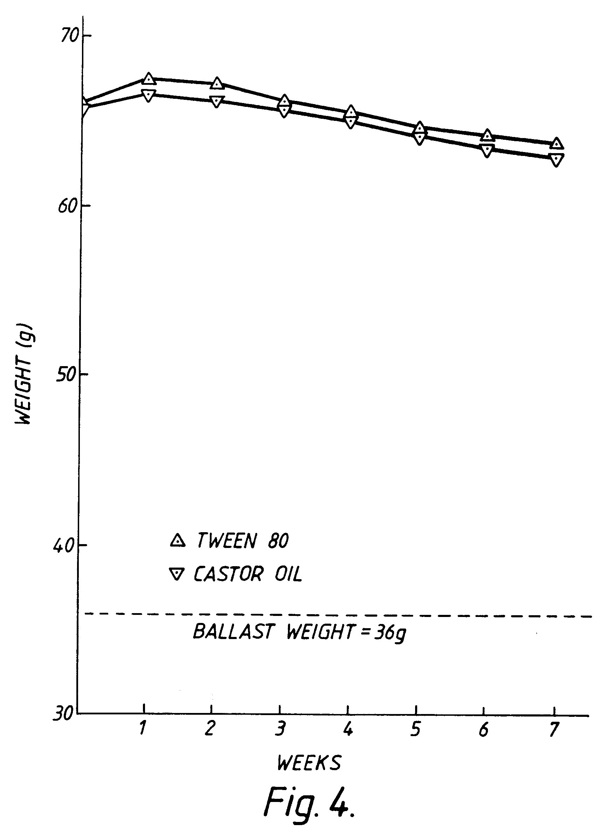

The boluses were each inserted into the reticulum of a fistulated cow. Figure 4 shows the loss of weight of the boluses within the cow over a period of several weeks. The erosion rate is fairly constant at about 0.2 grammes per day for both resin types.

Example 8

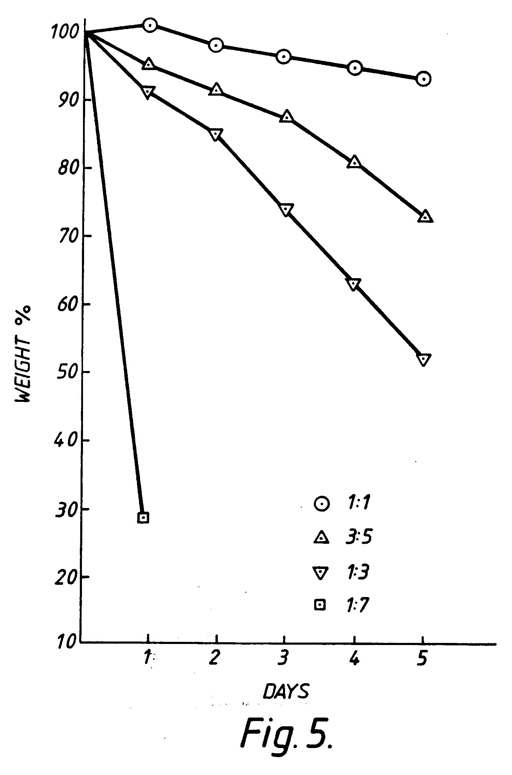

Four resin mixes were prepared from Epikote 828, DET and a non-bonding Polyetheylene glycol 400 Dioleate. The ratio of bonding:non-bonding material in the resins was 1:1, 3:5, 1:3 and 1:7 respectively. Four boluses were prepared by mixing 3 grammes of resin with 30 grammes of the same glass used in Example 7. 10 gramme quantities of each moulding mixture were pressed at 1-tonne to give specimens in the form of 25 mm discs. Each of the discs was subjected to a R.A.T.. The results of the R.A.T. are shown in figure 5. As the proportion of epoxide resin is reduced the composites become more susceptible to wet abrasion.

Example 9

Boluses were prepared in accordance with the method of Example 2 using three different glass grain size ranges, less than 75 um, between 75 and 150 um and greater than 150 um. These glasses were formed into boluses in accordance with the method of Example 2 with epoxide contents of 50 wt%, 37.5 wt% and 25 wt%. The boluses were then subjected to a R.A.T. The erosion rates in grammes per day are shown in Table 3.

## Table 3

| GRAIN SIZE (um) | wt% EPOXIDE | | |
| --- | --- | --- | --- |
| | 50 | 37.5 | 25 |
| 75 | 0.1 | 0.3 | 1.2 |
| 75 - 150 | 2.1 | 9.1 | * |
| 150 | 0.9 | * | * |

*Gone in 24 hours

Example 10

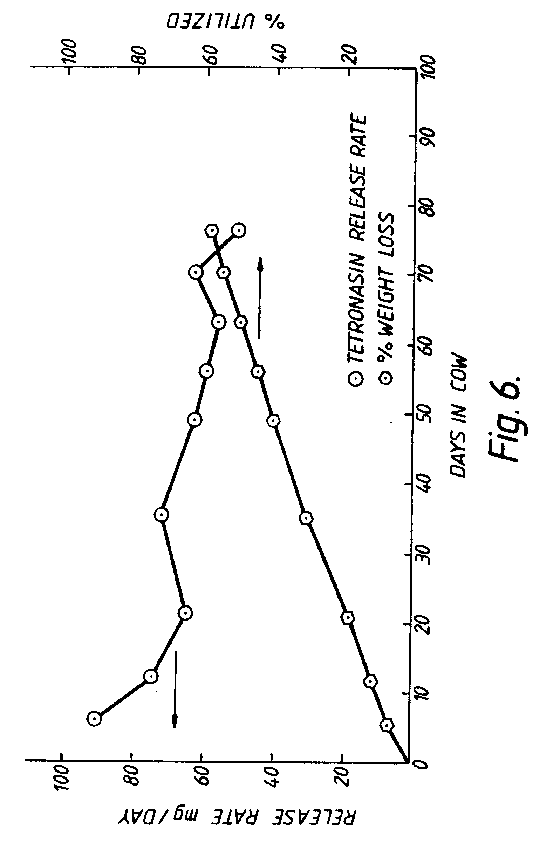

A bolus was prepared in accordance with the method of Example 2 and with Tween 20 substituted for Tween 80. The bolus contained Tetronasin as the active drug. The resin contained 73.2% Tween 20, 24.4% Epikote 828 and 2.4% DET. 9.1 parts of the resin were mixed with 65.9 parts of silicate window glass ground to a size of less than 200 micrometers and 25 parts of the Tetronasin drug of particle size less than 45 micrometers. Before pressing around the ballast weight the mixture had a density of 1.64 g/cm$^3$. When pressed around the ballast weight and cured the density rose to 6.5 g/cm$^3$ and the bolus weighed 40.64g. Pairs of such boluses were administered to cows and figure 6 shows the average percentage weight loss of the boluses and

6

EP 0 410 664 B1

the drug release rate. The graph shows a steady weight loss which gives rise to a slowly decreasing but within limits controlled release rate of the drug in a form in which it is available to the cow.

Example 11

The previous examples have used drugs which are naturally strongly hydrophobic and therefore tend to discourage the entry of water into the bulk of the bolus which could cause premature disintegration. A number of experiments were carried out to determine whether use of a hydrophobic silane coating on the glass could provide the same effect and therefore allow devices according to the invention to be used for release of less hydrophobic active materials. Resins were made up as for example 10 and a second batch of resin was produced with castor oil substituted for Tween 20. The moulding mix was 3 parts resin to 20 parts of window glass which had been ground to a powder and passed through a 200 micrometre sieve. Boluses were fabricated both from untreated glass powder and from glass coated with a monolayer of octadecyl trichrorosilane. Table 4 shows the weight of water absorbed by 10g boluses after 65 hours immersion. It is clear that the silane coting reduces the uptake of water both for the castor oil and the Tween 20 boluses. The water uptake in the case of castor oil is very low indeed and would allow the bolus to be loaded with drugs that were not hydrophobic.

Table 4

| Binder | Epoxy/Castor Oil | | Epoxy Tween | |
|---|---|---|---|---|
| Coating | None | Silane | None | Silane |
| Density g/cm | 1.87 | 1.97 | 1.81 | 2.05 |
| Void Space ml | 1.68 | 1.24 | 1.93 | 1.01 |
| Water Absorbed g | 0.45 | 0.03 | 1.02 | 0.27 |
| Voids Filled % | 27 | 2 | 53 | 27 |

**Claims**

1.  A composite bolus for sustained release of a sparingly soluble organic active material in the reticulo-rumen of an animal characterised in that it is produced by mixing together an adhesive comprising one or more insoluble thermosetting resins, which are highly substantive to glass when cured, with an equal or greater weight of a dispersion medium comprising one or more hydrophilic organic liquids which are miscible with or dissolve the uncured adhesive and phase separate from the adhesive when it is cured, to form a liquid binder; mixing the binder so formed with glass or other inorganic particles and particles of sparingly soluble active material to form a cake, the ratio of binder to particles being selected to ensure the presence of voids in the cake; and curing the adhesive to phase separate it and bond together the glass particles to form a rigid bolus.

2.  A composite bolus according to claim 1 in which the cake is pressed around a ballast weight before cure.

3.  A composite bolus according to claim 1 in which the cured cake is fixed to the ballast weight.

4.  A composite bolus according to any preceding claim in which the glass is durable glass.

5.  A composite bolus according to claim 4 in which the durable glass is 'E' glass.

6.  A composite bolus according to claim 4 in which the durable glass is soda-lime glass.

7.  A composite bolus according to any one of claims 1 to 3 in which the glass is a phosphate glass.

7

8. A composite bolus according to claim 7 in which the phosphate glass has a $P_2O_5$ content of greater than 50 wt% and an $Al_2O_3$ content of greater than 5 wt%.

9. A composite bolus according to any one of claims 1 to 4 in which the glass particle size is less than 200 micrometres and the average particle size of active substance is approximately 45 micrometres.

10. A composite bolus according to any preceding claims in which the adhesive comprises one or.more epoxy resins.

11. A composite bolus according to claim 10 in which one of the epoxy resins comprises bisphenol diglycidyl ether cured with an aliphatic polyamine.

12. A composite bolus according to claim 11 in which the aliphatic polyamine is diethylene triamine.

13. A composite bolus according to any preceding claim in which the organic liquid includes a liquid which is immiscible with water.

14. A composite bolus according to claim 13 in which the immiscible liquid is castor oil.

15. A composite bolus according to any one of claims 1 to 12 in which the organic liquid includes a liquid which is miscible with water.

16. A composite bolus according to claim 15 in which the miscible liquid is polyethylene glycol (1000) sorbitan mono-oleate or polyethylene glycol (1000) sorbitan mono-laurate.

17. A composite bolus according to any preceding claim in which the glass is coated with a silane to make the surface hydrophobic.

18. A method for the sustained supply of an active substance to a ruminant animal comprising administering simultaneously two or more boluses prepared in accordance with claim 1 to the reticulum of the animal, with the proviso that therapeutical treatments are excluded.

19. A method according to claim 18 in which two boluses are so administered.

20. A method according to claim 18 or claim 19 in which the boluses are of equal size and weight.

21. Use of a composite bolus according to any one of claims 1 to 17 as a delivery device for delivering an active material.

22. Use of a composite bolus according to claim 21 wherein the active material in a dietoxy supplement.

**Patentansprüche**

1. Zusammengesetzter Bolus zur verzögerten Freisetzung eines schwer löslichen organischen Wirkstoffs an den Netzmagen/Pansen eines Tieres, dadurch gekennzeichnet, daß er hergestellt worden ist durch Vermischen eines Klebemittels, umfassend ein oder mehrere unlösliche heißhärtende Harze, die, wenn sie gehärtet sind, gegenüber Glas sehr substantiv sind, mit einem gleichen oder größeren Gewichtsanteil eines Dispersionsmediums, umfassend eine oder mehrere hydrophile organische Flüssigkeiten, die mit dem nicht gehärteten Klebemittel mischbar sind oder dieses lösen und zu einer Phasentrennung führen, wenn das Klebemittel gehärtet ist unter Bildung eines flüssigen Bindemittels; Vermischen des so hergestellten Bindemittels mit Glas oder anderen anorganischen Teilchen und Teilchen von schwer lösbarem Wirkstoff unter Bildung eines Kuchens, wobei das Verhältnis von Bindemittel zu Teilchen so gewählt wird, um das Vorhandensein von Hohlräumen in dem Kuchen sicherzustellen, und Härten des Klebemittels, um eine Phasentrennung herbeizuführen und die Glasteilchen unter Bildung eines festen Bolus miteinander zu verbinden.

2. Zusammengesetzter Bolus nach Anspruch 1, wobei der Kuchen vor dem Härten um ein Ballastgewicht herum gepreßt worden ist.

3.  Zusammengesetzter Bolus nach Anspruch 1, bei dem der gehärtete Kuchen an dem Ballastgewicht fixiert ist.

4.  Zusammengesetzter Bolus nach einem der vorangehenden Ansprüche, bei dem das Glas ein haltbares bzw. beständiges Glas ist.

5.  Zusammengesetzter Bolus nach Anspruch 4, wobei das beständige Glas E-Glas ist.

6.  Zusammengesetzter Bolus nach Anspruch 4, wobei das beständige Glas ein Natronkalk-Glas ist.

7.  Zusammengesetzter Bolus nach einem der Ansprüche 1 bis 3, wobei das Glas ein Phosphatglas ist.

8.  Zusammengesetzter Bolus nach Anspruch 7, wobei das Phosphatglas einen $P_2O_5$-Gehalt größer 50 Gew.-% und einen $Al_2O_3$-Gehalt größer 5 Gew.-% aufweist.

9.  Zusammengesetzter Bolus nach einem der Ansprüche 1 bis 4, bei dem die Größe der Glasteilchen weniger als 200 μm und die mittlere Teilchengröße des Wirkstoffs etwa 45 μm beträgt.

10. Zusammengesetzter Bolus nach einem der vorangehenden Ansprüche, bei dem das Klebemittel ein oder mehrere Epoxyharze umfaßt.

11. Zusammengesetzter Bolus nach Anspruch 10, bei dem eines der Epoxyharze Bisphenol-diglycidylether, gehärtet mit einem aliphatischen Polyamin, umfaßt.

12. Zusammengesetzter Bolus nach Anspruch 11, bei dem das aliphatische Polyamin Diethylentriamin ist.

13. Zusammengesetzter Bolus nach einem der vorangehenden Ansprüche, bei dem die organische Flüssigkeit eine Flüssigkeit umfaßt, die mit Wasser nicht mischbar ist.

14. Zusammengesetzter Bolus nach Anspruch 13, bei dem die mit Wasser nicht mischbare Flüssigkeit Rizinusöl ist.

15. Zusammengesetzter Bolus nach einem der Ansprüche 1 bis 12, bei dem die organische Flüssigkeit eine Flüssigkeit umfaßt, die mit Wasser mischbar ist.

16. Zusammengesetzter Bolus nach Anspruch 15, wobei die mischbare Flüssigkeit Polyethylenglykol-(1000)-sorbitan-monooleat oder Polyethylenglykol-(1000)-sorbitan-monolaurat ist.

17. Zusammengesetzter Bolus nach einem der vorangehenden Ansprüche, bei dem das Glas mit einem Silan überzogen ist, um die Oberfläche hydrophob zu machen.

18. Verfahren zur verzögerten Abgabe eines Wirkstoffs an den Pansen eines Tieres, umfassend die gleichzeitige Verabreichung von zwei oder mehreren Boli, die hergestellt worden sind nach Anspruch 1, an den Netzmagen des Tieres, mit der Maßgabe, daß therapeutische Behandlungen ausgeschlossen sind.

19. Verfahren nach Anspruch 18, bei dem zwei Boli so verabreicht werden.

20. Verfahren nach Anspruch 18 oder 19, wobei die Boli in Größe und Gewicht gleich sind.

21. Verwendung eines zusammengesetzten Bolus nach einem der Ansprüche 1 bis 17 als Abgabevorrichtung zur Abgabe eines Wirkstoffs.

22. Verwendung eines zusammengesetzten Bolus nach Anspruch 21, wobei der Wirkstoff ein Nahrungsmittelzusatz ist.

**Revendications**

1.  Bolus composite pour la libération entretenue d'une substance organique active moyennement soluble dans le réticulo-rumen d'un animal, caractérisé en ce qu'il est préparé en mélangeant ensemble un adhésif comprenant une ou plusieurs résines thermodurcissables insolubles, qui adhèrent fortement au verre lorsqu'elles sont prises, avec un poids égal ou supérieur d'un milieu de dispersion comprenant un ou plusieurs liquides organiques hydrophiles qui sont miscibles avec l'adhésif non durci ou le dissolvent et forment une phase séparée de l'adhésif lorsque ce dernier est pris, pour former un liant liquide ; en mélangeant le liant ainsi formé avec des particules de verre ou d'autres particules inorganiques et des particules d'une substance active moyennement soluble pour former un gâteau, le rapport du liant aux particules étant choisi pour assurer la présence de vides dans le gâteau ; et en faisant prendre l'adhésif pour le former en phase séparée et lier ensemble les particules de verre pour former un bolus rigide.

2.  Bolus composite selon la revendication 1, dans lequel le gâteau est pressé autour d'un ballast avant la prise.

3.  Bolus composite selon la revendication 1, dans lequel le gâteau durci est fixé sur le ballast.

4.  Bolus composite selon la revendication 1, dans lequel le verre est un verre durable.

5.  Bolus composite selon la revendication 4, dans lequel le verre durable est un verre "E".

6.  Bolus composite selon la revendication 4, dans lequel le verre durable est un verre à la chaux-soude.

7.  Bolus composite selon l'une des revendications 1 à 3, dans lequel le verre est un verre au phosphate.

8.  Bolus composite selon la revendication 7, dans lequel le verre au phosphate a une teneur en $P_2O_5$ supérieure à 50% en poids et une teneur en $Al_2O_3$ supérieure à 5% en poids.

9.  Bolus composite selon l'une des revendications 1 à 4, dans lequel la dimension des particules de verre est inférieure à 200 $\mu$m et la dimension moyenne des particules de substance active est environ 45 $\mu$m.

10. Bolus composite selon l'une des revendications précédentes, dans lequel l'adhésif comprend une ou plusieurs résines époxy.

11. Bolus composite selon la revendication 10, dans lequel l'une des résines époxy contient un éther diglycidylique de biphénol traité avec une polyamine aliphatique.

12. Bolus composite selon la revendication 11, dans lequel la polyamine aliphatique est la diéthylène triamine.

13. Bolus composite selon l'une des revendications précédentes, dans lequel le liquide organique contient un liquide qui n'est pas miscible à l'eau.

14. Bolus composite selon la revendication 13, dans lequel le liquide non miscible est l'huile de ricin.

15. Bolus composite selon l'une des revendications 1 à 12, dans lequel le liquide organique contient un liquide qui est miscible à l'eau.

16. Bolus composite selon la revendication 15, dans lequel le liquide miscible est le mono-oléate de polyéthylène glycol (1000) sorbitanne ou le mono-laurate de polyéthylène glycol (1000) sorbitanne.

17. Bolus composite selon l'une des revendications précédentes, dans lequel le verre est revêtu d'un silane pour rendre sa surface hydrophobe.

18. Méthode pour la fourniture entretenue d'une substance active à un animal ruminant, consistant à administrer simultanément deux ou plusieurs bolus préparés selon la revendication 1 dans le réseau (réticulum) de l'animal, sous réserve que sont exclus des traitements thérapeutiques.

19. Méthode selon la revendication 18, dans laquelle deux bolus sont ainsi administrés.

20. Méthode selon l'une des revendications 18 ou 19, dans laquelle les bolus ont la même dimension et le même poids.

21. Utilisation d'un bolus composite selon l'une des revendications 1 à 17 comme dispositif de fourniture pour délivrer une substance active.

22. Utilisation d'un bolus composite selon la revendication 21, dans laquelle la substance active est un complément alimentaire.

*Fig.1.*

Fig. 2.

Fig. 3.

Fig. 4.

Fig. 5.

Fig. 6.